# EUROPEAN PATENT SPECIFICATION

(11) **EP 2 496 271 B1**
(45) Date of publication and mention of the grant of the patent: **28.05.2014**
(21) Application number: 10776127.2
(22) Date of filing: 03.11.2010
(51) Int. Cl.: A61L 2/10, A61L 9/20, A61L 2/24, A61L 2/28

(54) **STERILISING UV EMITTER**
UV-STERILISATIONSEMITTER
ÉMETTEUR UV DE STÉRILISATION

(30) Priority: 04.11.2009 GB 0919333
(43) Date of publication of application: 12.09.2012
(73) Proprietor: Nanoclave Technologies LLP, London W1S 3NX (GB)
(72) Inventor: FUDAKOWSKI, Adam, London W1T 6QD (GB); JAMES, John, Rugby Warwickshire CV22 7EN (GB); ROBERT, Charles, London WV11 2VU (GB)
(74) Representative: Sanger, Phillip Simon
(86) International application number: PCT/GB2010/051835
(87) International publication number: WO 2011/055140

(56) References cited:
- EP-A1- 1 346 735
- EP-A1- 1 602 878
- WO-A1-01/60419
- WO-A1-95/17634
- CN-Y- 2 155 875

## Description

The present invention is concerned with a sterilising emitter. In particular, the present invention is concerned with a portable sterilising lamp having multiple operable configurations for transport and deployment.

It is known in the art that ultra-violet light has a sterilising effect. Consequently, UV light has been used to sterilise medical equipment and materials. Such sterilisation is effected by exposure of the devices or materials to ultra-violet light. Devices such as hoods and containers containing UV lamps allow a user to place equipment therein in order to bombard that equipment with ultra-violet radiation. This bombardment results in a germicidal effect, sterilising the equipment therein.

A problem with known systems is that decontamination of entire rooms using ultra-violet light requires either systematic coverage of the entire surface of a room with a hand-held device or, alternatively, the use of a very large UV device which is expensive and bulky necessitating transport to and from the room between uses.

A known UV emitter is disclosed in EP1346735A1.

It is an aim of the present invention to provide an improved sterilizing emitter.

According to a first aspect of the invention there is provided a sterilising emitter according to claim 1.

Advantageously, provision of two relatively moveable UV emitters allows the sterilising emitter to be put into a transport condition whereby the second UV emitter is retracted. The second emitter can be moved to its extended or deployed position wherein it provides significant UV coverage in conjunction with the first emitter. Therefore, the advantages of a large emitter are realised with the portability of a smaller device. Furthermore, even when the second emitter is in the retracted position, the first emitter is still capable of emitting UV radiation effectively because it is at least partially exposed. This allows the device to be operated when in its transport condition and thus offers the flexibility of use of a much smaller emitter whilst having the potential coverage of a much larger device.

Preferably, in the retracted position of the second UV emitter, the first UV emitter is independently operable to emit UV radiation.

Advantageously, the first UV emitter can therefore provide UV coverage without using the second emitter at all. Advantageously, this saves power when the second emitter is in the retracted position.

Preferably, the second UV emitter is slidably moveable parallel to a main axis of the first UV emitter, and even more preferably the first and second UV emitters are telescopic.

This provides a very compact construction for transportation of the device in the transport condition (i.e. when the second emitter is in the retracted position).

Preferably, a third UV emitter is provided movable relative to the second UV emitter from a retracted position to a deployed position. More preferably, in the retracted position of the UV emitter, the first and second UV emitters are operable independent of the third UV emitter to emit UV radiation.

Advantageously, the arrangement of three UV emitters provides even more flexibility of the device such that it can be operated in the transport condition (with the first UV emitter exposed), an intermediate condition (in which the first and second UV emitters are exposed) and in the fully deployed condition (in which all emitters are simultaneously exposed).

This provides flexibility depending on the size and dimensions of the room or area the user wishes to decontaminate.

Preferably at least one satellite sub-emitter is provided, configured to be movable relative to the emitter and being controlled by the emitter.

Preferably a remote UV meter is provided having a data connection to the emitter, which remote UV meter is arranged to provide UV exposure feedback to the emitter. This allows the emitter to sense when sterilisation is complete.

Preferably a diffuser is provided, comprising a material configured to scatter UV light.

An example UV emitter and method according to the present invention will now be described with reference to the accompanying figures in which:
FIGURE la is a side view of a first sterilising emitter in accordance with the present invention in a transport condition;
FIGURE 1b is a side view of the sterilising emitter of Figure 1a in an intermediate position;
FIGURE 1c is a side view of the sterilising emitter of Figure 1a in a fully deployed position;
FIGURE 2 is a side view of the sterilising emitter of Figure 1a comprising a transport cover;
FIGURE 3a is a plan view of a second sterilising emitter in accordance with the present invention;
FIGURE 3b is a schematic side view of the sterilising emitter of Figure 3a in a transport condition;
FIGURE 3c is a schematic side view of the sterilising emitter of Figure 3a in an intermediate position;
FIGURE 3d is a side view of the sterilising emitter of 3a in a fully deployed condition;
FIGURE 4 is a side view of a third sterilising emitter in accordance with the present invention;
FIGURE 5 is a side view of a fourth sterilising emitter in accordance with the present invention, and;
FIGURE 6 is a side view of a fifth sterilising emitter in accordance with the present invention.

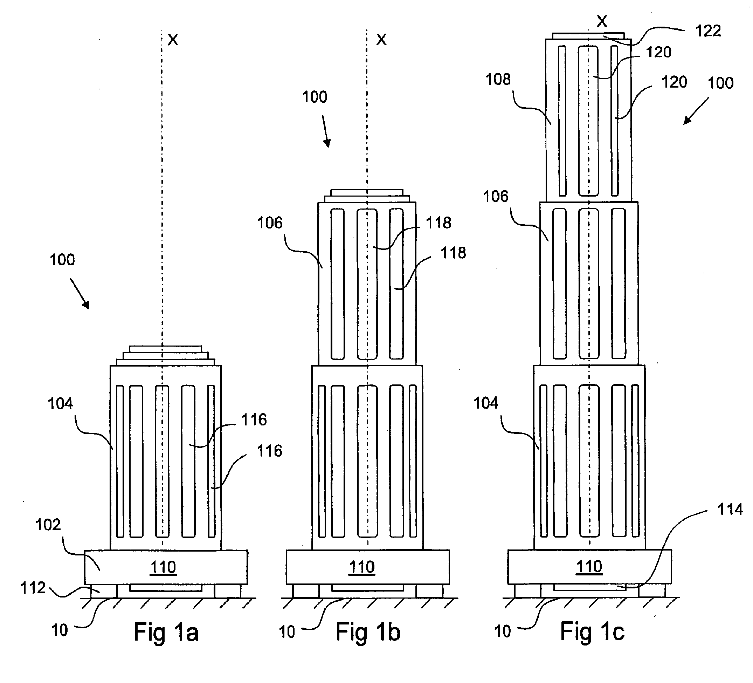

Referring to Figures 1a to 1c a sterilising emitter 100 is shown. The emitter 100 comprises a base 102, a first emitter section 104, a second emitter section 106 and a third emitter section 108.

The base 102 rests on the ground 10 and comprises a casing 110 having a plurality of feet 112 circumferentially spaced about the periphery thereof. The feet 112 hold the casing 110 spaced away from the ground.

The base 102 further comprises a downwardly facing base UV lamp 114 which is arranged to project UV radiation away from the underside of the casing 110 onto the ground 10. The first emitter section 104 is cylindrical and tubular and is connected to the base casing 110. The first emitter section 104 defines a main axis of the sterilising emitter X. The first emitter section 104 comprises a plurality of ultra-violet emitting strip lamps 116 aligned parallel to the axis X. The ultra-violet emitting strip lamps 116 are positioned at equal circumferential positions around the first emitter section 104 such that when activated, ultra-violet light is emitted across a range of 360 degrees from the first emitter section 104.

Referring to Figure 1b, the second emitter section 106 is similar to the first emitter section 104. The second emitter section 106 is cylindrical, tubular and has a series of ultra-violet emitting strip lamps 118 positioned circumferentially thereabout. The diameter of the second emitter section 106 is less than the diameter of the first emitter section 104 such that the second emitter section 16 can be positioned telescopically within the first emitter section 104 as shown in Figure 1a.

Referring to Figure 1c, the third emitter section 108 is similar to the first and second emitter sections 104, 106 having a plurality of ultra-violet emitting strip lamps 120 spaced circumferentially thereabout. The third emitter section 108 is diametrically smaller than the second emitter section 106 such that it can be positioned telescopically within the second emitter section 106 as shown in Figure 1. The third emitter section 108 comprises an axial emitter 122 which is arranged to project ultra-violet radiation along the axis X away from the third axial section 108.

As can be seen by comparing Figures 1a, 1b and 1c, the second and third emitter sections 106, 108 can be nested within each other and within the first emitter section 104. In this position, all three emitter sections overlap and only the UV emitting strip lamps 116 of the first emitter section 104 are outwardly exposed to emit UV radiation radially. In the condition as shown in Figure 1a (the transport condition), the UV emitting strip lamps 116 of the first section 104 can be operated independent of the UV emitter strip lamps 118, 120 such that the emitter 100 in this condition is useable without wasting any energy by illuminating the concealed lamps 118, 120.

In the intermediate condition shown in 1b, the second emitter section 106 has been moved to a deployed condition in which the UV emitting strip lamps 118 are exposed. In this intermediate position both the UV emitting strip lamps 116 and the lamps 118 can be operated together and independently of the lamps 120.

When in the fully deployed condition shown in Figure 1c, all three sets of lamps can be operated together providing significant coverage.

It will be noted that the ability to independently activate each of the three sets of lamps may be provided by the provision of three independent switches on the base 102.

Alternatively, a single switch may be provided on the base 102 and the illumination of the lamps 118, 120 determined by the position of the sections 106, 108. A microswitch or similar device may be provided between the sections 104, 106 such that when the second emitter section 106 is in the deployed condition as shown in Figures 1b and 1c, activation of the switch will cause both the lamps 116 and 118 to illuminate. Similarly, in the position shown in Figure 1c, all three sets of lamps will illuminate. In the position shown in Figure 1a, because both the sections 106, 108 are retracted, activation of the switch will only operate the lamps 116 of the first section 104. The sections 106, 108 are unable to be activated when in their retracted positions.

The operational details of circuits to provide this function is known and as such will not be described further here.

In order to provide more UV coverage, both the base UV lamp 114 and the axial lamp 122 will be activated regardless of the condition of the sterilising emitter 100.

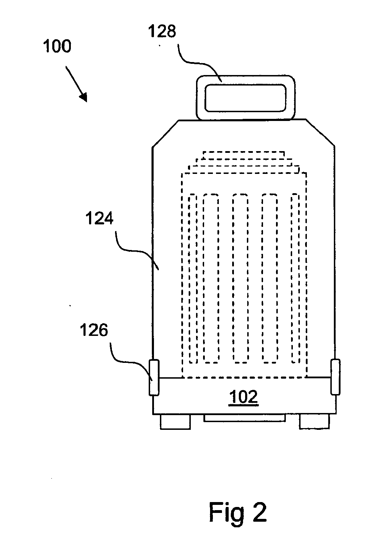

Referring to Figure 2, the sterilising emitter 100 is shown, covered by a cover 124 attached to the base 102 via clips 126. The cover 124 comprises a handle 128 for carrying of the emitter 100.

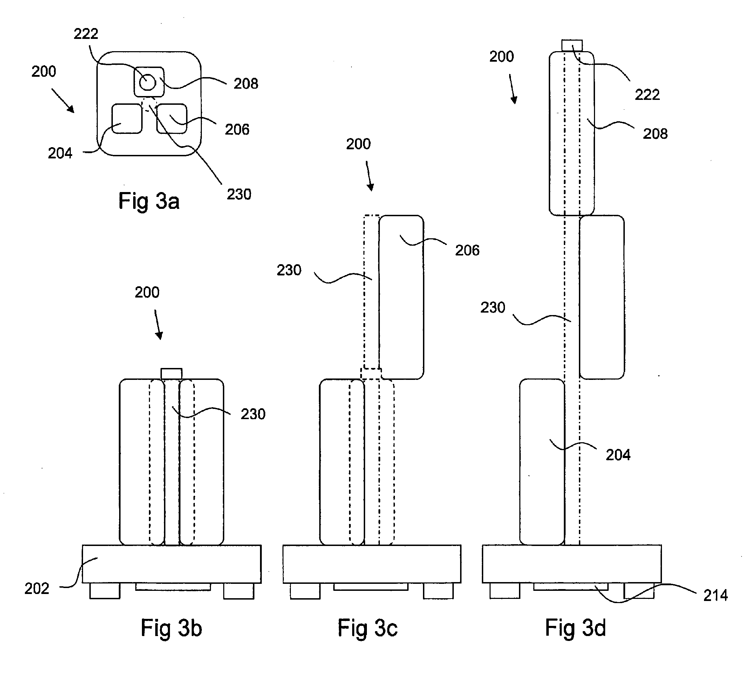

Referring to Figures 3a to 3c, these figures show a schematic representation of an alternative sterilising emitter configuration 200.

The sterilising emitter 200 comprises a base 202 similar to the base 102. A telescopic arrangement is shown schematically at 230. The telescopic arrangement 230 has three emitter sections 204, 206 and 208 attached thereto. The emitter sections 204, 206, 208 all comprise UV emitting lamps. A downwardly facing base UV lamp 214 and an axial emitter 222 are provided similar to the emitter 100.

The telescopic arrangement 230 is configured such that the emitter sections 204, 206, 208 can be overlapped as shown in Figure 3b, the second emitter section 206 can be extended as shown in Figure 3c and the third emitter section 208 can be extended above the second emitter section as shown in Figure 3d. The exact details of the telescopic arrangement 230 will not be described here but are known. It will be understood that the arrangement 230 is minimal in construction to avoid the casting of significant shadows.

Unlike the sterilising emitter 100, when the sterilising emitter 200 is in the transport condition all three emitter sections 204, 206, 208 can be simultaneously activated to provide 360 degree protection. Alternatively, only one of the sections (e.g. the first emitter section 104), could be activated, and the telescopic arrangement 230 could be rotatably attached to the base 202 via a carousel arrangement such that the emitter 204 is rotated and thus achieves 360 degree UV coverage.

Such an arrangement would also be beneficial in the intermediate condition of Figure 3c and the fully deployed condition of Figure 3d such that 360 degree emission is obtained eliminating any problems of the telescopic arrangement 230 blocking emitted UV radiation from any one of the emitter sections.

It will be understood that both the emitters 208 and 206 may be positioned in the intermediate position of 206 as shown in figure 3c. This may be useful if extra radiation is required at an intermediate height. Under these circumstances the emitter 208 can be activated in this position as well as its fully deployed position per figure 3d.

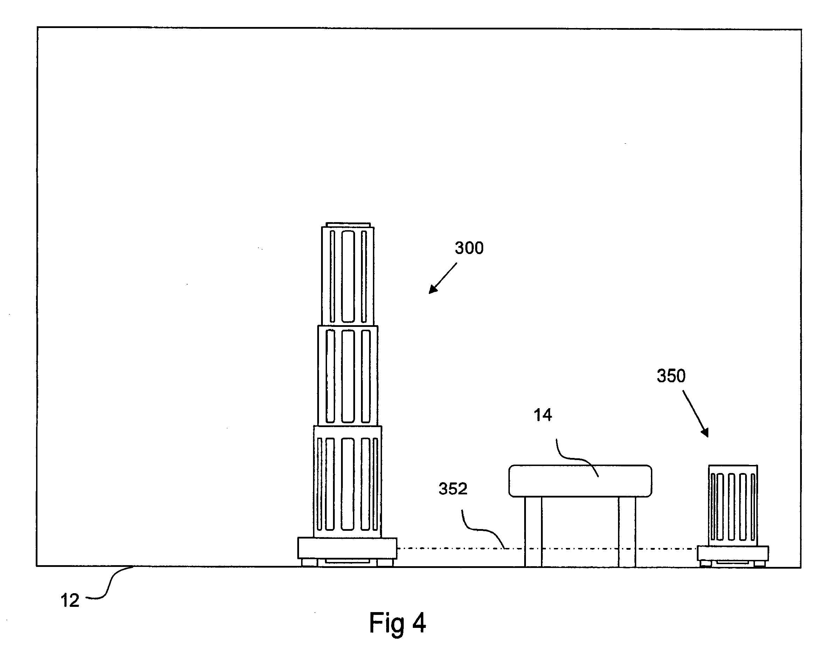

Turning to Figure 4, an emitter 300 is shown which is similar to emitter 100. The emitter 300 is shown within a room to be sterilised 12 containing a bed 14. The emitter 300 comprises a satellite sub-emitter 350 connected to the emitter 300 via a combined data and power link 352. The sub-emitter 350 is arranged to expose areas of the room 12 and bed 14 which would otherwise not be exposed to UV-radiation due to shadowing, in particular from the bed 14.

Any number of sub-emitters may be provided, and it is envisaged that they are controlled by the emitter 300 to provide a single point of user interaction.

In a further embodiment (not shown), the sub-emitter or emitters 350 may be stored in a casing of the emitter 300 to provide a compact and portable arrangement.

Optionally, the sub-emitters 350 may have their own, independent power source (such as a battery or mains adapter) and be controlled by the emitter via a data link. Optionally, the data link may be wireless (e.g. bluetooth, radio link).

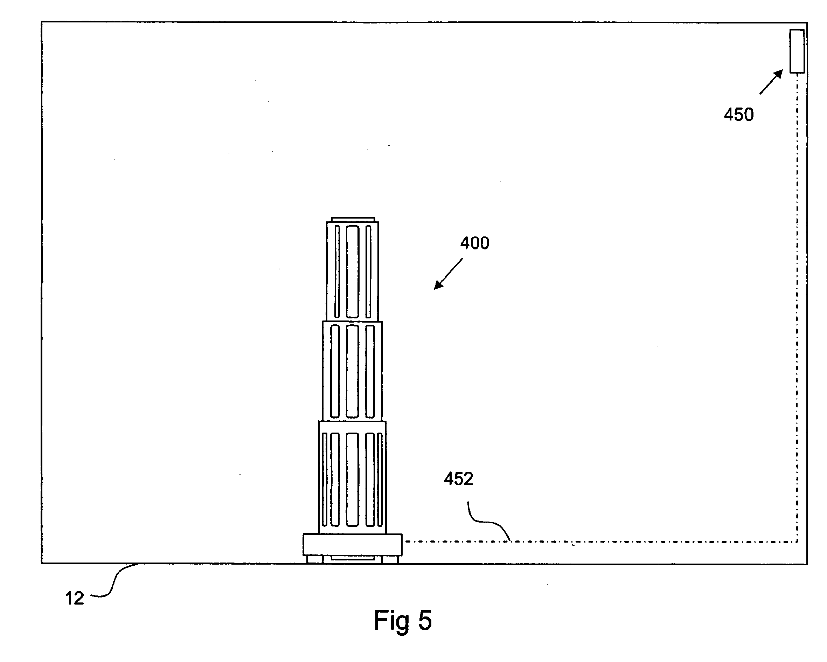

Turning to figure 5, an emitter 400 is shown which is similar to emitter 100. The emitter 400 is shown within a room to be sterilised 12. The emitter 400 comprises a remote UV-meter 450 connected to the emitter 400 via a combined data and power link 452. The remote UV-meter 450 is positioned in a part of the room to be decontaminated 12 which is likely to receive the lowest UV coverage. The UV-meter 450 will provide feedback to a user or to a control system to determine when desired UV exposure has occurred in the lowest UV area. Therefore the user can be confident that the area has been successfully decontaminated. Such meters may be connected to the emitter 100 by a wired or wireless link.

Optionally, a plurality of detectors may be used, the lowest UV exposure reading used to determine the point at which the room or area has been sufficiently sterilised. Optionally, the meter or meters may be stowed in the casing of the emitter 400 for storage and transportation.

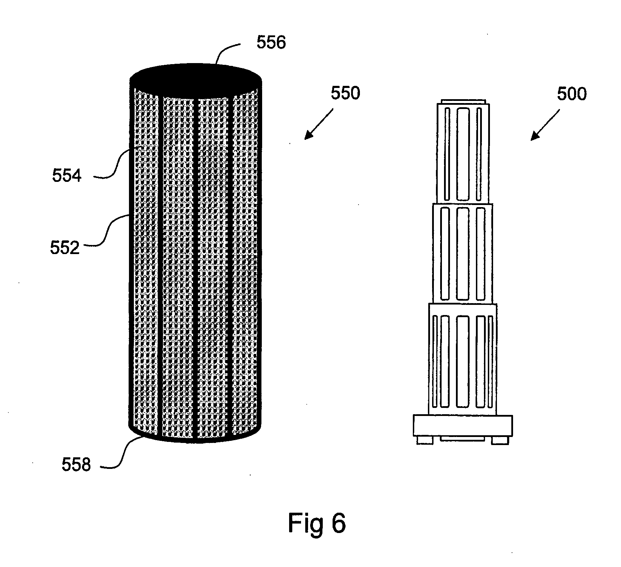

Turning to figure 6, an emitter 500 is shown which is similar to emitter 100. The emitter 500 is provided with a diffuser 550 which comprises a cylindrical frame 552 in which a diffuser material 554 is mounted. The diffuser is sealed at a top end 556 with the material 554 and open at a bottom end 558 so it can be placed over the emitter 500.

The diffuser material may be a fabric such as teflon (RTM) or quartz fibre and is of an appropriate thickness to be at least partially transparent to UV light. As the UV light passes through the fabric, it is scattered. Scattered UV light is more effective at sterilising surfaces because bacterial shadowing is minimised (i.e. where bacteria are positioned on top of one another).

The diffuser 550 may be collabible for storage and transport.

It will be understood that variations may fall within the scope of the present invention.

The features of the above examples may be combined. For example an emitter may have a satellite emitter, a UV-meter and / or a diffuser.

The emitter may have two, four or more emitter sections. The UV emission means mounted to each emitter section need not be strip lamps, but may be LED's, laser or any other type of lamp or device capable of emitting UV or sterilising radiation.

The UV emitter sections of Figures 1a to 1c may be attached to the base 102 via a carousel as well to provide a rotating 360 degree sweep of ultra-violet emission. Alternately a smaller angle reciprocating sweep may be used to ensure than shadows resulting from any components blocking the UV lamps do not cause "blind spots".

The emitter 100 may comprise a motion detector to detect when a person is present in the vicinity of the emitter 100, and automatically deactivate. This prevents unintentional exposure of users to the emitter 100. The motion detector may be provided in the base 102. The motion detector may comprise a passive infra-red detector or a camera.

The emitter 100 may be activated and deactivated via a remote control, which may be wired or wireless. Therefore the user need not be proximate the device when it is activated and therefore exposure to UV radiation is minimised. The remote control may also comprise a fault indicator (e.g. a lamp) to indicate whether any of the UV lamps are malfunctioning. The indicator preferably functions only when the sections 104, 106, 108 are deactivated. Alternatively, the emitter 100 may comprises a delay switch which only activates the emitter after a specified time interval to allow the user to leave the room.

## Claims

1. A sterilising emitter (100; 200; 300; 400; 500) comprising;
a first UV emitter (104; 204), and,
a second UV emitter (106; 206),
**characterised in that** the second UV emitter (106; 206) is slideably movable relative to the first UV emitter (104; 204) from a retracted position in which the first and second UV emitters at least partially overlap to a deployed position, wherein in the retracted position the first UV emitter is at least partially exposed to emit UV radiation.

2. A sterilising emitter (100; 200; 300; 400; 500) according to claim 1 in which in the retracted position of the second UV emitter the first UV emitter and the second UV emitter are adjacent.

3. A sterilising emitter (100; 200; 300; 400; 500) according to claim 1 or 2 in which in the retracted position of the second UV emitter the first UV emitter is independently operable to emit UV radiation.

4. A sterilising emitter (100; 200; 300; 400; 500) according to any preceding claim in which the second UV emitter is slideably movable along a main axis (X) of the first UV emitter.

5. A sterilising emitter (100; 300; 400; 500) according to claim 4 in which the first and second UV emitters are telescopic.

6. A sterilising emitter (100; 200; 300; 400; 500) according to any preceding claim comprising a third UV emitter (108; 208) movable relative to the second UV emitter from a retracted position to a deployed position.

7. A sterilising emitter (100; 200; 300; 400; 500) according to claim 6 comprising an axial emitter (122; 222) arranged on the third emitter to project UV radiation substantially along the main axis (X) away from the third emitter.

8. A sterilising emitter (100; 200; 300; 400; 500) according to any preceding claim comprising a base (102; 202) connected to the first emitter (104; 204) to permit substantially vertical orientation of the UV emitters when the base is rested on a substantially horizontal surface.

9. A sterilising emitter (100; 200; 300; 400; 500) according to claim 8 in which the base comprises a base UV emitter (114; 214) arranged to emit UV radiation from a side of the base opposite the first UV emitter.

10. A sterilising emitter (100; 200; 300; 400; 500) according to claim 9 in which the first UV emitter is connected to the base (114; 214) via a carousel arranged to cause relative rotation of the base and first UV emitter.

11. A sterilising emitter (100; 200; 300; 400; 500) according to any of claims 8 to 10 in which the base comprises a power source.

12. A sterilising emitter (100; 200; 300; 400; 500) according to any preceding claim in which the second and/or third emitters are operable in their respective deployed conditions only.

13. A sterilising emitter (300) according to any preceding claim comprising at least one satellite sub-emitter (350) configured to be movable relative to the emitter and being controlled by the emitter.

14. A sterilising emitter (400) according to any preceding claim comprising a remote UV meter (450) having a data connection to the emitter, which remote UV meter is arranged to provide UV exposure feedback to the emitter.

15. A sterilising emitter (500) according to any preceding claim comprising a diffuser (550) comprising a material configured to scatter UV light.

## Patentansprüche

1. Sterilisationsemitter (100; 200; 300; 400; 500), der umfasst:
einen ersten UV-Emitter (104; 204) und
einen zweiten UV-Emitter (106; 206),
**dadurch gekennzeichnet, dass** der zweite UV-Emitter (106; 206) relativ zu dem ersten UV-Emitter (104; 204) aus einer eingefahrenen Position, in der der erste und der zweite UV-Emitter wenigstens teilweise überlappen, in eine ausgefahrene Position gleitend beweglich ist, wobei in der eingefahrenen Position der erste UV-Emitter wenigstens teilweise freiliegt, um UV-Strahlung zu emittieren.

2. Sterilisationsemitter (100; 200; 300; 400; 500) nach Anspruch 1, wobei in der eingefahrenen Position des zweiten UV-Emitters der erste UV-Emitter und der zweite UV-Emitter zueinander benachbart sind.

3. Sterilisationsemitter (100; 200; 300; 400; 500) nach Anspruch 1 oder 2, wobei in der eingefahrenen Position des zweiten UV-Emitters der erste UV-Emitter unabhängig betreibbar ist, um UV-Strahlung zu emittieren.

4. Sterilisationsemitter (100; 200; 300; 400; 500) nach einem vorhergehenden Anspruch, wobei der zweite UV-Emitter längs einer Hauptachse (X) des ersten UV-Emitters gleitend beweglich ist.

5. Sterilisationsemitter (100; 300; 400; 500) nach Anspruch 4, wobei der erste und der zweite UV-Emitter teleskopartig sind.

6. Sterilisationsemitter (100; 200; 300; 400; 500) nach einem vorhergehenden Anspruch, der einen dritten UV-Emitter (108; 208) umfasst, der relativ zu dem zweiten UV-Emitter aus einer eingefahrenen Position in eine ausgefahrene Position beweglich ist.

7. Sterilisationsemitter (100; 200; 300; 400; 500) nach Anspruch 6, der einen axialen Emitter (122; 222) umfasst, der an dem dritten Emitter angeordnet ist, um UV-Strahlung im Wesentlichen längs der Hauptachse (X) weg von dem dritten Emitter zu projizieren.

8. Sterilisationsemitter (100; 200; 300; 400; 500) nach einem vorhergehenden Anspruch, der eine Basis (102; 202) umfasst, die mit dem ersten Emitter (104; 204) verbunden ist, um eine im Wesentlichen vertikale Orientierung der UV-Emitter zu ermöglichen, wenn die Basis auf einer im Wesentlichen horizontalen Oberfläche ruht.

9. Sterilisationsemitter (100; 200; 300; 400; 500) nach Anspruch 8, wobei die Basis einen Basis-UV-Emitter (114; 214) umfasst, der dafür ausgelegt ist, UV-Strahlung von einer Seite der Basis gegenüber dem ersten UV-Emitter zu emittieren.

10. Sterilisationsemitter (100; 200; 300; 400; 500) nach Anspruch 9, wobei der erste UV-Emitter mit der Basis (114; 214) über ein Karussell verbunden ist, das dafür ausgelegt ist, eine relative Drehung der Basis und des ersten UV-Emitters zu bewirken.

11. Sterilisationsemitter (100; 200; 300; 400; 500) nach einem der Ansprüche 8 bis 10, wobei die Basis eine Leistungsquelle enthält.

12. Sterilisationsemitter (100; 200; 300; 400; 500) nach einem vorhergehenden Anspruch, wobei der zweite und/oder der dritte Emitter nur in ihren jeweiligen ausgefahrenen Zuständen betreibbar sind.

13. Sterilisationsemitter (300) nach einem vorhergehenden Anspruch, der wenigstens einen Satelliten-Unteremitter (350) umfasst, der konfiguriert ist, relativ zu dem Emitter beweglich zu sein und durch den Emitter gesteuert zu werden.

14. Sterilisationsemitter (400) nach einem vorhergehenden Anspruch, der ein entferntes UV-Messgerät (450) umfasst, das eine Datenverbindung mit dem Emitter besitzt, wobei das entfernte UV-Messgerät dafür ausgelegt ist, eine Rückmeldung der UV-Exposition für den Emitter bereitzustellen.

15. Sterilisationsemitter (500) nach einem vorhergehenden Anspruch, der einen Diffusor (550) umfasst, der ein Material enthält, das konfiguriert ist, UV-Licht zu streuen.

## Revendications

1. Emetteur de stérilisation (100 ; 200 ; 300 ; 400 ; 500) comprenant :
un premier émetteur d'UV (104 ; 204), et
un deuxième émetteur d'UV (106 ; 206),
**caractérisé en ce que** le deuxième émetteur d'UV (106 ; 206) est mobile de manière coulissante par rapport au premier émetteur d'UV (104 ; 204) à partir d'une position rétractée dans laquelle les premier et deuxième émetteurs d'UV se recouvrent au moins en partie, jusqu'à une position déployée, étant précisé que dans la position rétractée, le premier émetteur d'UV est au moins en partie dégagé pour émettre un rayonnement UV.

2. Emetteur de stérilisation (100 ; 200 ; 300 ; 400 ; 500) selon la revendication 1, dans lequel dans la position rétractée du deuxième émetteur d'UV, le premier émetteur d'UV et le deuxième émetteur d'UV sont adjacents.

3. Emetteur de stérilisation (100 ; 200 ; 300 ; 400 ; 500) selon la revendication 1 ou 2, dans lequel dans la position rétractée du deuxième émetteur d'UV, le premier émetteur d'UV est apte à fonctionner indépendamment pour émettre un rayonnement UV.

4. Emetteur de stérilisation (100 ; 200 ; 300 ; 400 ; 500) selon l'une quelconque des revendications précédentes, dans lequel le deuxième émetteur d'UV est mobile de manière coulissante le long d'un axe principal (X) du premier émetteur d'UV.

5. Emetteur de stérilisation (100 ; 200 ; 300 ; 400 ; 500) selon la revendication 4, dans lequel les premier et deuxième émetteurs d'UV sont télescopiques.

6. Emetteur de stérilisation (100 ; 200 ; 300 ; 400 ; 500) selon l'une quelconque des revendications précédentes, comprenant un troisième émetteur d'UV (108 ; 208) qui est mobile par rapport au deuxième émetteur d'UV, à partir d'une position rétractée jusqu'à une position déployée.

7. Emetteur de stérilisation (100 ; 200 ; 300 ; 400 ; 500) selon la revendication 6, comprenant un émetteur axial (122 ; 222) qui est disposé sur le troisième émetteur pour projeter un rayonnement UV globalement le long de l'axe principal (X) à partir du troisième émetteur.

8. Emetteur de stérilisation (100 ; 200 ; 300 ; 400 ; 500) selon l'une quelconque des revendications précédentes, comprenant une base (102 ; 202) reliée au premier émetteur (104 ; 204) pour permettre une orientation globalement verticale des émetteurs d'UV quand la base est posée sur une surface globalement horizontale.

9. Emetteur de stérilisation (100 ; 200 ; 300 ; 400 ; 500) selon la revendication 8, dans lequel la base comprend un émetteur d'UV de base (114 ; 214) qui est conçu pour émettre un rayonnement UV à partir d'un côté de la base opposé au premier émetteur d'UV.

10. Emetteur de stérilisation (100 ; 200 ; 300 ; 400 ; 500) selon la revendication 9, dans lequel le premier émetteur d'UV est relié à la base (114 ; 214) par l'intermédiaire d'un carrousel conçu pour provoquer une rotation relative de la base et du premier émetteur d'UV.

11. Emetteur de stérilisation (100 ; 200 ; 300 ; 400 ; 500) selon l'une quelconque des revendications 8 à 10, dans lequel la base comprend une source d'alimentation.

12. Emetteur de stérilisation (100 ; 200 ; 300 ; 400 ; 500) selon l'une quelconque des revendications précédentes, dans lequel les deuxième et/ou troisième émetteurs ne sont aptes à fonctionner que dans leurs conditions déployées respectives.

13. Emetteur de stérilisation (100 ; 200 ; 300 ; 400 ; 500) selon l'une quelconque des revendications précédentes, comprenant au moins un sous-émetteur satellite (350) qui est conçu pour être mobile par rapport à l'émetteur et qui est commandé par l'émetteur.

14. Emetteur de stérilisation (100 ; 200 ; 300 ; 400 ; 500) selon l'une quelconque des revendications précédentes, comprenant un radiomètre UV distant (450) qui présente une connexion de données avec l'émetteur, ledit radiomètre UV distant étant conçu pour fournir à l'émetteur un retour d'information d'exposition.

15. Emetteur de stérilisation (100 ; 200 ; 300 ; 400 ; 500) selon l'une quelconque des revendications précédentes, comprenant un diffuseur (550) comprenant un matériau conçu pour disperser la lumière UV.
